# EUROPÄISCHE PATENTSCHRIFT

(11) **EP 1 266 874 B1**
(45) Veröffentlichungstag und Bekanntmachung des Hinweises auf die Patenterteilung: **22.09.2004**
(21) Anmeldenummer: 02090214.4
(22) Anmeldetag: 14.06.2002
(51) Int. Cl.: C03C 10/16, A61L 27/12, A61L 27/32

(54) **Glasig-kristallines Material mit geringer Löslichkeit und Verfahren zu seiner Herstellung**
Vitreous-crystalline material of low solubility and method of its preparation
Matière vitreuse-cristalline de faible solubilité et son procédé de réalisation

(30) Priorität: 15.06.2001 DE 10129844; 17.05.2002 DE 10223102
(43) Veröffentlichungstag der Anmeldung: 18.12.2002
(73) Patentinhaber: BUNDESANSTALT FÜR MATERIALFORSCHUNG UND -PRUFUNG (BAM), D-12205 Berlin (DE)
(72) Erfinder: Berger, Georg, Dr., 16341 Zepernick (DE); Ploska, Ute, Dr., 12433 Berlin (DE)
(74) Vertreter: Walter, Wolf-Jürgen

(56) Entgegenhaltungen:
- US-A- 4 820 660
- US-A- 5 077 132
- US-A- 5 232 878
- US-A- 6 013 591

## Beschreibung

Die Erfindung betrifft ein glasig-kristallines Material mit geringer Löslichkeit, das sowohl als bioaktiver Knochenersatzwerkstoff, so z.B. als Beschichtung von metallischen Prothesenstielen durch thermisches Spritzen, als auch als Substratmaterial in der Biotechnologie, z.B. als keramische Folie oder Formkörper Anwendung finden kann. Die Erfindung betrifft auch ein Herstellungsverfahren.

Anorganische Materialien mit Langzeitstabilität sind an sich bekannt. Auch Werkstoffe, die ihren speziellen Einsatz als bioaktive Knochenersatzwerkstoffe finden und eine ausreichende Langzeitstabilität besitzen, sind in der Literatur beschrieben. Beispielsweise wurde ständig über den erfolgreichen klinischen Einsatz von Glaskeramiken bzw. Sinterglaskeramiken mit den Hauptkristallphasen Apatit und Wollastonit berichtet [Kokubo, T., Biomaterials, 12 (1991)155 - 163; Berger, G. et al.: Long-term stable bioactive glass ceramics an implant material - ten years of clinical experience, Forth World Biomaterial Congress, Berlin, April 24 - 28,1992, Transactions p. 33]. übertroffen wurde deren chemische Stabilität durch ebenfalls bioaktive Werkstoffe auf der Basis von Calcium-Zirconium/Titan-Phosphat (Biomaterials 18 (1997) 1671 - 1675), die sich nur durch keramische Verfahren herstellen lassen, jedoch keine Schmelze bei in der Glasindustrie üblichen Temperaturen ( etwa 1650°C) bilden, was bekanntermaßen Nachteile bei der mechanischen Stabilität von derartigen Granulaten und insbesondere daraus hergestellten Formkörpern bewirkt.

Der Erfindung liegt die Aufgabe zugrunde, ein glasig-kristallines Material bereitzustellen, das einen direkten, bindegewebsfreien Knochenverbund ermöglicht und dennoch chemisch langzeitstabil ist.

Erfindungsgemäß besteht das glasig-kristalline Material auf Basis von CaO, P₂O₅, ZrO₂ und Fluorid aus 15 - 45 Gew-% CaO, 40 - 45 Gew-% P₂O₅, 10 - 40 Gew-% ZrO₂ und 0,7 - 3,5 Gew-% Fluorid und enthält als Hauptkristallphasen Apatit und Calciumzirconiumphosphat und als Nebenbestandteil eine Glasphase, wobei die Hauptkristallphasen zusammen wenigstens 35 Gew-% betragen und die Nebenbestandteile 5 bis 15 Gew-% betragen, und wobei alle Prozentangaben auf das Gesamtgewicht des glasig-kristallinen Materials bezogen sind.

Ein bevorzugtes glasig-kristallines Material enthält 23 - 39 Gew-% CaO, 40 - 45 Gew-% P₂O₅, 20 - 35 Gew-% ZrO₂ und 1 - 3 Gew-% Fluorid und enthält als Hauptkristallphasen Apatit und Calciumzirconiumphosphat und als Nebenbestandteil eine Glasphase, wobei die Hauptkristallphasen zusammen wenigstens 35 Gew-% betragen und die Nebenbestandteile 5 bis 15 Gew-% betragen.

Ein ebenfalls bevorzugtes glasig-kristallines Material enthält 20 - 35 Gew-% CaO, 40 - 45 Gew-% P₂O₅, 20 - 35 Gew-% ZrO₂ und 1 - 3 Gew-% Fluorid sowie zusätzlich 0,1 bis 6 Gew-% Na₂O, und es enthält als Hauptkristallphasen Apatit und Calciumzirconiumphosphat und als Nebenbestandteil eine Glasphase und zusätzlich als Nebenbestandteil eine Natriumzirconiumphosphat-Phase.

Dabei beträgt der Anteil der Hauptkristallphasen zusammen wenigstens 35 Gew-%, und die Nebenbestandteile können jeweils 5 bis 15 Gew-% betragen.

Weiterhin kann das erfindungsgemäße glasig-kristalline Material zusätzlich 0,1 bis 6 Gew-% Magnesiumoxid und/oder Kaliumoxid enthalten und gegebenenfalls auch die entsprechenden Phasen als Nebenbestandteile.

Der Gehalt an Na₂O, MgO und/oder K₂O liegt vorzugsweise im Bereich von 1 bis 6 Gew-%. Der Anteil der entsprechenden Nebenkristallphase Natriumzirconiumphosphat liegt vorzugsweise im Bereich von 5 bis 10 Gew-%.

In einer vorteilhaften Ausführungsform können in das zu schmelzende Gemenge bis zu 15 Gew-% eines gemahlenen Glases eingebracht werden, das aus SiO₂, Al₂O₃ und MgO und gegebenenfalls CaO besteht und in seiner Zusammensetzung und seinen Eigenschaften einem Cordierit-Glas entspricht, wodurch die Sinterfähigkeit des erfindungsgemäßen glasig-kristallinen Materials deutlich verbessert wird. Ein solches zugesetzes Glas ist besonders hochschmelzend (> 1500°C) und chemisch stabil gegen Wasser, Säuren und Laugen.

Die hier verwendeten Begriffe "Glaskeramik" und "glasig-kristallines Material" sind im Allgemeinen nicht immer eindeutig definierbar. Es liegen sowohl kristalline als auch glasige bzw. röntgenamorphe Phasen innig vermischt vor. Für die vorliegende Erfindung ist es ohne Belang, ob eine Phase neben der anderen vorliegt oder ob eine Phase die andere umhüllt.
Als "Hauptkristallphase" wird hier eine kristalline Phase bezeichnet, deren Mengenanteil wenigstens doppelt so groß ist, wie die einer Nebenphase, wobei Konzentrationen um 15 Gew-% und darunter, vorzugsweise unter 10 Gew-% als Nebenphasen bezeichnet werden.

überraschend wurde nun gefunden, daß der Werkstoff, obwohl er Apatit enthält, auch im leicht sauren Medium, wie es bei Entzündungsreaktionen beobachtet wird, nämlich pH = 6,0 [Berger et al., Hydroxyapatite's solubility may cause loosening of coated implants, Bioceramics Vol. 13, edited by Santro Giannini and Antonio Moroni (Proceedings of the 13^{th} International Symposium on Ceramics in Medicine); Trans Tech Publ. Ltd, Swiss, 2000, 111 - 114], sehr lösungsstabil ist.

Ferner wurde überraschenderweise gefunden, daß nach Lagerung in deionisiertem Wasser der Werkstoff (das glasig-kristalline Material) nach anfänglich alkalischer Reaktion seine Oberflächeneigenschaften in Richtung physiologische pH-Werte (7,4) zu verändert, was ihn auch für die Biotechnologie interessant macht.

Daß eine Verarbeitung des erfindungsgemäßen Werkstoffes zu geeigneten Schlickern zur Herstellung von spongiosa-artigen Formkörpern und keramischen Folien überraschenderweise möglich ist, kann man zunächst ebenfalls nicht erwarten, da es hinreichende Beispiele von in der Literatur bekannten Werkstoffen gibt, die diese Eigenschaften nicht aufweisen.

Der thermische Ausdehnungskoeffizient des neuen Materials liegt zwischen 27°C und 300, 400, 600 oder 800°C im Bereich von 1,4 und 6•10⁻⁶ grad⁻¹. Er liegt im Bereich von 1,4 und 8 •10⁻⁶ grad⁻¹ zwischen 27°C und 300, 400, 600 und 800 °C, wenn die Herstellung des Materials über Haltestufen beim Abkühlen der Schmelze erfolgt, wie weiter unten beschrieben.

Ein weiteres Merkmal des Materials besteht darin, daß es eine Gesamtlösung von 4 bis 5,5 mg/L hat, wenn die Prüfung in 0,2M TRIS-HCl-Puffer-Lösung bei pH=7,4, T=37°C, an einer Kornfraktion von 315 - 400µm, 120h lang bei einem Oberflächen(Probe)-zu-Volumen(Lösungsmittel)-Verhältnis von 5cm⁻¹ erfolgt.

Ein weiteres Merkmal des Materials besteht darin, daß es bereits nach Wasserlagerung (144h) bei 37 °C die Oberfläche des Werkstoffes so einstellt, daß physiologische pH-Werte um 7,4 bestimmt werden können. Erhöht man die Wasserbadtemperatur, so wird die Oberflächenveränderung dementsprechend beschleunigt herbeigeführt.

Wenn in einer weiter unten beschriebenen Ausführungsform bei der Herstellung des erfindungsgemäßen Werkstoffes ein oder mehrere Haltestufen beim Abkühlen der Schmelze im Ofen zwischen 800 und 1100 °C eingelegt werden, können Glaskeramiken mit folgenden Eigenschaften erhalten werden:
- Gesamtlösung von 0,2 bis 2,0 mg/L, wenn die Prüfung in 0,2M TRIS-HCl-Puffer-Lösung bei pH 7,4, T=37°C, an einer Kornfraktion von 315 - 400 µm, 120h lang bei einem Oberflächen(Probe)-zu-Volumen(Lösungsmittel)-Verhältnis von 5cm⁻¹ erfolgt,
- thermischer Ausdehnungskoeffizient zwischen 1,4 und 8•10⁻⁶ grad⁻¹ zwischen 27°C und 300, 400, 600 und 800 °C
- Stabilität im pH-Bereich zwischen 7,0 und 7,5. Bei einem solchen Material liegt somit die chemische Beständigkeit um den Faktor 3 - 10 höher als die des Materials ohne Haltestufen, da die Gesamtlösung im Bereich von 0,2 - 2,0 mg/l liegt.

Erfindungsgemäß wird der Werkstoff hergestellt, indem die für die Gemengebildung geeignete Substanzen 15 - 45 Gew-% CaO, 40 - 45 Gew-% P₂O₅, 10 - 40 Gew-% ZrO₂ und 0,7 - 3,5 Gew-% Fluorid kombiniert werden, und in geeigneten meist mehrstufigen Temperaturbehandlungsprogrammen (Haltestufen im Bereich von 400 bis 1500 °C) in einem geeigneten Tiegelmaterial, zum Beispiel bestehend aus einer Pt/Rh-Legierung, bei 1550 bis 1650 °C zum Schmelzen gebracht werden. Die Schmelze wird vergossen, und die erstarrte Schmelze wird je nach Verwendungszweck an der Luft (spontane Abkühlung) oder im Kühlofen auf Raumtemperatur abgekühlt, gegebenenfalls unter Einhaltung von Haltestufen.

Haltestufen im Temperaturbehandlungsprogramm von jeweils ca. 1h bei 400, 800 und 1000 °C können die Reproduzierbarkeit der Einschmelzreaktion verbessern.

Das Fluorid wird vorteilhaft als CaF₂ eingebracht mit Konzentrationen von 1,5 - 7 Gew-%. Es kann auch als ZrF₂ oder gegebenenfalls als NaF, KF oder MgF₂ eingebracht werden.

In einer weiteren Ausführungsform ist das Verfahren dadurch gekennzeichnet, daß das genannte Gemenge aus 15 - 45 Gew-% CaO, 40-45 Gew-% P₂O₅, 10 - 40 Gew-% ZrO₂ und 0,7 - 3,5 Gew-% Fluorid mit mehreren Haltestufen im Bereich von 400 bis 1500°C bei 1550 bis 1650°C aufgeschmolzen und kontrolliert im Ofen mit einer Geschwindigkeit von 60°C/h bis 300°C/h mit zwei Haltestufen zwischen 1000 bis 1100°C und 800 bis 1000°C für 2 bis 10 h abgekühlt wird.

Nach der Abkühlung wird der Werkstoff beispielsweise aufgemahlen, mit üblichen Sinterhilfsmitteln versetzt und sodann zu Formkörpern verpreßt, um nach dem Sintern einen möglichst dicht gebrannten keramischen Scherben zu erhalten.

Der erfindungsgemäß hergestellte Werkstoff kann auch beispielsweise aufgemahlen, mit üblichen Sinterhilfsmitteln versetzt und zu Schlicker verarbeitet werden, dieser auf einen Polyurethanschwamm aufgebracht und in mehreren Sinterstufen so hoch gesintert werden, daß der Polyurethanschwamm und die Sinterhilfsmittel ausgebrannt werden und ein spongiosa-artiger Formkörper mit den kristallinen Hauptbestandteilen an Apatit und Calciumzirconiumphosphat erhalten wird.

Eine weitere Verarbeitungsmöglichkeit besteht darin, den Werkstoff aufzumahlen, mit üblichen Sinterhilfsmitteln zu versetzen und den so erhaltenen Schlicker zu einer Folie zu verarbeiten, die nach dem Brennprozeß eine offenporige Struktur vorweist.

Die Verwendung des erfindungsgemäßen glasig-kristallinen Materials zur Herstellung von Granulaten, keramischen Formkörpern oder keramischen Folien ist ebenfalls Gegenstand der Erfindung.

Die Erfindung wird nachstehend durch Beispiele näher erläutert. Alle Prozentangaben sind auf das Gewicht bezogen, sofern nicht anderes angegeben ist.

In der dazugehörigen Zeichnung zeigen
- Fig. 1: Röntgendiffraktogramm des Materials von Beispiel 1
- Fig. 2: Röntgendiffraktogramm des Materials von Beispiel 2.

### Beispiel 1

Es wird ein Gemenge bereitet, das folgender Zusammensetzung entspricht (Code: Apatit/CZP1) :
25,88 CaO
28,44 ZrO₂
43,68 P₂O₅
5,00 CaF₂

Dabei erweist es sich als praktikabel, den CaO-Anteil in Form von 62,79 CaHPO₄ einzusetzen und den noch notwendigen P₂O₅-Anteil in Form von 10,51ml einer 85%igen H₃PO₄. Zunächst wird CaHPO₄, ZrO₂ und CaF₂ gut vermischt, danach mit der Phosphorsäure versetzt, nach Reaktion gemörsert und in einen Trockenschrank gebracht. Dort werden insgesamt 4h Trockenhaltestufen im Bereich von 120°C und 170°C eingelegt. Das Reaktionsgemisch wird entnommen und in einen Pt/Rh-Tiegel gefüllt und über die 1h Haltestufen 400 und 800°C erhitzt, abgekühlt und anschließend gemörsert. Das so vorbehandelte Material wird nunmehr im Pt/Rh-Tiegel mit jeweils 15min Haltezeit in den Stufen 800, 1000, 1300, 1500 und schließlich 1600°C zum Schmelzen gebracht und sodann auf eine Stahlplatte (Raumtemperatur) gegossen.

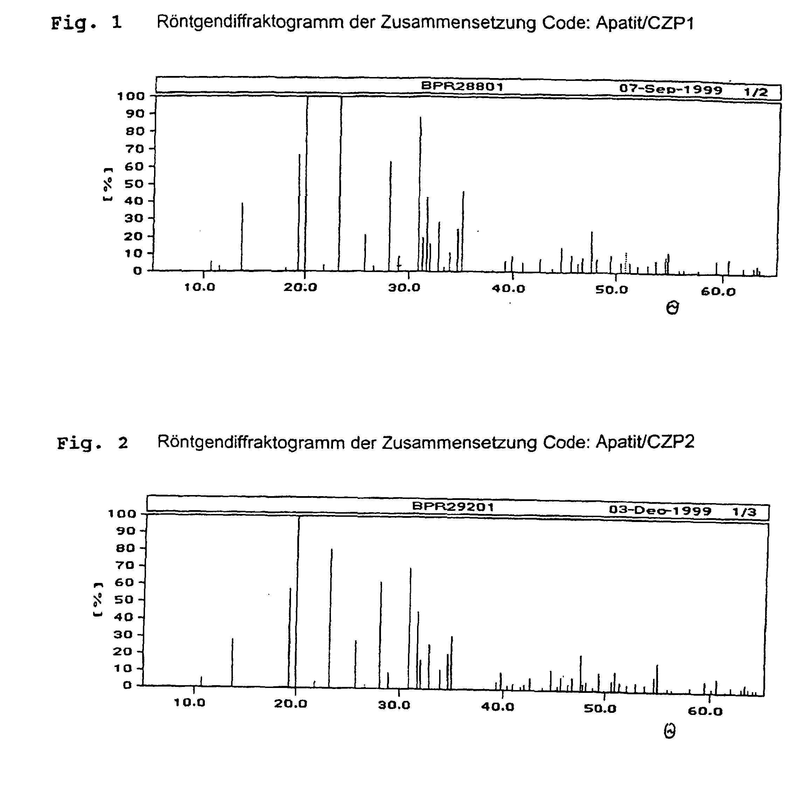

Ein Teil der abgekühlten Schmelze wurde durch Mahlen in einer Achatmühle zerkleinert, unter 43µm abgesiebt und sodann einer röntgendiffraktographischen Untersuchung unterzogen. Das Ergebnis (vergl. Fig. 1) zeigt, daß in dem glasig-kristallinen Produkt die Kristallphasen Apatit (Fluorapatit/Hydroxylapatit) und Calciumzirconiumphosphat [CaZr₄(PO₄)₆] deutlich nachweisbar sind.

### Beispiel 2

Es wird ein Gemenge nach der Vorschrift des Beispiels 1 hergestellt mit dem Unterschied, daß hier als zusätzliche Komponente Natriumoxid eingebracht wird (Code: Apatit/CZP2). Der Ansatz sieht die Verwendung folgender Gemengebestandteile vor:
59,93 CaHPO₄
27,10 ZrO₂
3,42 Na₂O
5,00 CaF₂ und
9,56ml einer 85%igen H₃PO₄-Säure.

Es wurde wie im Beispiel 1 gearbeitet. Nach der letzten Temperaturhaltestufe wurde die Schmelze aus dem Tiegel auf eine Stahlplatte gegossen.
Ein Teil der Schmelze wurde durch Mahlen in einer Achatmühle zerkleinert, unter 43µm abgesiebt und sodann einer röntgendiffraktographischen Untersuchung unterzogen. Das Ergebnis (vergl. Fig. 2) zeigt, das in dem glasig-kristallinen Produkt die Kristallphasen Apatit (Fluorapatit/Hydroxylapatit) und Calciumzirconiumphosphat
[CaZr₄(PO₄)₆] und Natriumzirconiumphosphat [NaZr₂(PO₄)₃] nachweisbar sind.

### Beispiel 3

Es erfolgte die Herstellung eines glasig-kristallinen Materials nach Beispiel 1 (Apatit/CZP1). Das Material wird durch Mahlen in einer mit Zirconiumoxid ausgekleideten Mühle zerkleinert, so daß sich ein D₅₀-Wert von 8µm ergab. Das gemahlene Gut wird mit einer 5%igen Polyvinylalkohol(PVA)-Lösung im Verhältnis Mahlgut zu PVA-Lösung von 90 : 10 Masse-% versetzt und zu einem Stab mit 4,7kN in einem Pressgesenk verpreßt.

Dieser Rohkörper wird gesintert bei einer Temperatur von 1050°C.

An dem auf diese Weise erhaltenen relativ dichten Formkörper wird der thermische Ausdehnungskoeffizient(AK) bestimmt:
AK im Bereich von 27 - 400°C: 1,90*10⁻⁶ grd Celsius⁻¹
AK im Bereich von 50 - 400°C: 1,86*10⁻⁶ grd Celsius⁻¹
AK im Bereich von 30 - 300°C: 1,45*10⁻⁶ grd Celsius⁻¹
AK im Bereich von 30 - 400°C: 1,88*10⁻⁶ grd Celsius⁻¹
AK im Bereich von 30 - 600°C: 2,6*10⁻⁶ grd Celsius⁻¹
AK im Bereich von 30 - 800°C: 3,2*10⁻⁶ grd Celsius⁻¹

### Beispiel 4

Es erfolgt die Herstellung eines glasig-kristallinen Materials nach Beispiel 1 (Apatit/CZP1). Sodann wird das Material gemörsert und eine Kornfraktion von 315 - 400µm daraus hergestellt.

Das auf diese Weise erhaltene Granulat wird im Vergleich zu einem Grundglas (Ap40_{Glas}) und einer aus diesem Grundglas hergestellten Glaskeramik auf der Basis von Apatit und Wollastonit (Ap40_{krist.}) [chemische Zusammensetzung also identisch zu (Gewichts-%): 44,3 SiO₂; 11,3 P₂O₅; 31,9 CaO; 4,6 Na₂O; 0,19 K₂O; 2,82 MgO und 4,99 CaF₂, hergestellt gemäß Patent DD 247 574] hinsichtlich seiner chemischen Beständigkeit verglichen.

Zu diesem Zweck wurden zunächst die spezifischen Oberflächen nach BET mit Krypton als Meßgas bestimmt zu:
Apatit/CZP1: 0,364m²/g
Ap40_{Glas}: 0,018 m²/g
Ap40_{krist.}: 0,055m²/g.

Hier zeigt sich, daß das erfindungsgemäße Material eine gewisse offene Porosität im Vergleich zum Grundglas und der daraus hergestellten Glaskeramik aufweist. Diesen Unterschieden wird bei den Lösungsuntersuchungen dadurch Rechnung getragen, indem das Oberflächen(Proben) zu Lösungsmittelvolumen(TRIS-HCl-Puffer-Lösung) konstant auf 5cm⁻¹ eingestellt wird.

Als Lösungsmittel wurde eine 0,2M TRIS-HCl-Puffer-Lösung mit pH=7,4 bei 37°C verwendet. Die Lagerung erfolgte bei 37°C über eine Zeitdauer von 120h. Danach wurde die Gesamtlöslichkeit durch Bestimmung der Einzelionen (Ca, P, Zr) in der Lösung mit Hilfe einer ICP-Messung bestimmt zu:
Apatit/CZP1: 4,1 - 5,1 mg/L
Ap40_{Glas}: 318 - 320mg/L
Ap40_{krist.}: 75,2 - 82,0 mg/L.

Die Werte belegen eindrucksvoll die hohe chemische Beständigkeit des erfindungsgemäßen Werkstoffes unter simulierten physiologischen Bedingungen, einer bekannten Methode zur Bestimmung der Langzeitbeständigkeit in vitro.

### Beispiel 5

Es wird vorgegangen wie im Beispiel 4, jedoch wird eine 0,2M TRIS-HCl-Puffer-Lösung mit dem pH-Wert von 6,0 bei 37°C zur Messung verwendet. Auf diese Weise läßt sich der Fall simulieren, daß infolge einer Wundheilungs- oder Spätinfektion der pH-Wert von den physiologischen 7,4 in den sauren Bereich abgleitet.
Mit Hilfe der ICP wurden nun folgende Werte der Gesamtlösung (Ca, P, Zr) bestimmt:
Apatit/CZP1: 16-19 mg/L
Ap40_{Glas}: 505-518 mg/L
Ap40ₖᵣᵢₛₜ : 117-125 mg/L.

Die Werte belegen eindrucksvoll die hohe chemische Beständigkeit des erfindungsgemäßen Werkstoffes unter simulierten Bedingungen, wie sie bei einer Entzündungsreaktion vorliegen. Demzufolge ist die Erhöhung der absoluten Werte der Löslichkeit für den erfindungsgemäßen Werkstoff wesentlich geringer im Vergleich zu der doch dramatischen Erhöhung im Falle des Grundglases bzw. der Glaskeramik auf Apatit/Wollastonit-Basis.

### Beispiel 6

Es erfolgt die Herstellung eines glasig-kristallinen Materials nach Beispiel 2 (Apatit/CZP2). Das Material wird durch Mahlen in einer mit Zirconiumoxid ausgekleideten Mühle zerkleinert, so daß sich ein D₅₀-Wert von 8µm ergibt. Von diesem gemahlenen Gut werden 100g mit 45g eines Gemisches, bestehend aus 90 Gew-% Polyethylenglykol und 10 Gew-% eines handelsüblichen Netzmittels, unter Zugabe von 5ml Isopropylalkohol zu einem Schlicker versetzt. Dieser Schlicker wird auf PUR-Schwämme mit offener Porosität mit 80 bis 20 ppi (Poren pro Inch) durch mehrmaliges Eintauchen und Ausdrücken aufgebracht, im Trockenschrank über Nacht bei 120°C getrocknet und dann langsam mit 1°C pro Minute auf 1050°C erhitzt. Im Ergebnis ist ein spongiosa-artiges Material mit dem Ausgangsschwamm ähnlichem Aufbau vorhanden, und der PUR-Schwamm ist rückstandslos ausgebrannt.

### Beispiel 7

Es erfolgt die Herstellung eines glasig-kristallinen Materials nach Beispiel 1 (Apatit/CZP1). Das Material wird durch Mahlen in einer mit Zirconiumoxid ausgekleideten Mühle zerkleinert, so daß sich ein D₅₀-Wert von 8µm ergibt. Von diesem frisch gemahlenen Gut werden 1g in 100ml eines deinonisierten Wassers nach ISO 3696 eingebracht und die pH-Wert-Änderung über 144h gemessen.

Überraschenderweise wurde nun gefunden, daß der pH-Wert von 8,8 nach einer Stunde Lagerung in deionisierten Wasser nach 144h auf den physiologischen Wert von 7,4 zurückgeht, was den Werkstoff insbesondere für die Biotechnologie interessant macht.

### Beispiel 8

Es wird ein Gemenge nach der Vorschrift des Beispiels 1 hergestellt mit dem Unterschied, daß hier folgende Komponenten-Zusammensetzung gewählt wurde (Code: Apatit/CZP3) :
80,79 g CaHPO₄
19,42 g ZrO₂
4,87 g CaF₂ und
0,62 ml einer 85%igen Phosphorsäure.

Es wurde wie im Beispiel 1 gearbeitet mit dem Unterschied, daß die Schmelze nicht auf eine Stahlplatte gegossen wurde, sondern definiert im Ofen abkühlte mit jeweils zwei Haltestufen bei 1050°C (6h) und 950°C (6h). Die Gesamtlösung diese Materials beträgt 0,81 mg/l, wenn die Prüfung in 0,2M TRIS-HCl-Puffer-Lösung bei pH 7,4, T=37°C, an einer Kornfraktion von 315 - 400 µm, 120h lang bei einem Oberflächen(Probe)-zu-Volumen(Lösungsmittel)-Verhältnis von 5cm⁻¹ erfolgt.

### Beispiel 9

Es wird ein Gemenge nach der Vorschrift des Beispiels 8 (Apatit/CZP3) hergestellt wobei hier folgende Komponenten-Zusammensetzung zur Anwendung kam:

| | |
|---|---|
| 37,38 Gew.% | CaO |
| 14,45 Gew.% | ZrO₂ |
| 42,64 Gew.% | P₂O₅ |
| 5,53 Gew.% | CaF₂ |

Ferner wurde diesem gesinterten und gemahlenen Material zur Verbesserung der Sinterfähigkeit 10 Gew-% eines aufgemahlenen Glases (D₅₀ = 4,6 µm) der Zusammensetzung:

| | |
|---|---|
| 12,05 Gew.% | MgO |
| 1,00 Gew.% | CaO |
| 38,00 Gew.% | Al₂O₃ |
| 48,95 Gew.% | SiO₂ |

zugesetzt.

Sodann wurde dieses Materialgemisch nach Beispiel 6 weiterverarbeitet mit dem Unterschied, daß die Temperung wie im Folgenden dargestellt durchgeführt wurde:
Mit dem Schlicker benetzter PUR-Schwamm wurde mit 700°C/h auf 1300°C erhitzt, wobei als Ergebnis ein spongiosa-artiger Formkörper erhalten wurde.

## Patentansprüche

1. Glasig-kristallines Material mit geringer Löslichkeit auf Basis von CaO, P₂O₅, ZrO₂ und Fluorid, **dadurch gekennzeichnet, daß** es 15 - 45 Gew-% CaO, 40 - 45 Gew-% P₂O₅, 10 - 40 Gew-% ZrO₂ und 0,7 - 3,5 Gew-% Fluorid enthält und als Hauptkristallphasen Apatit und Calciumzirconiumphosphat und als Nebenbestandteil eine Glasphase enthält, wobei die Hauptkristallphasen zusammen wenigstens 35 Gew-% betragen und die Nebenbestandteile 5 bis 15 Gew-% betragen, und wobei alle Prozentangaben auf das Gesamtgewicht des glasig-kristallinen Materials bezogen sind.

2. Material nach Anspruch 1, **dadurch gekennzeichnet, daß** es 23 - 39 Gew-% CaO, 40 - 45 Gew-% P₂O₅, 20 - 35 Gew-% ZrO₂ und 1 - 3 Gew-% Fluorid enthält.

3. Material nach Anspruch 1 oder 2, **dadurch gekennzeichnet, daß** es zusätzlich 0,1 bis 6 Gew-% Na₂O, MgO und/oder K₂O enthält und entsprechende Phasen als zusätzlichen Nebenbestandteil.

4. Material nach Anspruch 1, **dadurch gekennzeichnet, daß** es zusätzlich eine Natriumzirconiumphosphat-Phase enthält.

5. Material nach Anspruch 1, **dadurch gekennzeichnet, daß** es als Granulat oder als keramisch verarbeiteter dichter oder poröser Formkörper oder als keramische Folie vorliegt.

6. Material nach Anspruch 1, **dadurch gekennzeichnet, daß** es eines oder mehrere der folgenden Parameter aufweist
- Gesamtlösung von 0,2 bis 5,5 mg/L, wenn die Prüfung in 0,2M TRIS-HCl-Puffer-Lösung bei pH=7,4, T=37°C, an einer Kornfraktion von 315 - 400µm, 120h lang bei einem Oberflächen(Probe)-zu-Volumen(Lösungsmittel)-Verhältnis von 5cm⁻¹ erfolgt,
- thermischer Ausdehnungskoeffizient zwischen 1,4 und 8•10⁻⁶ grad⁻¹ zwischen 27°C und 300, 400, 600 und 800 °C,
- Stabilität im pH-Bereich zwischen 7,0 und 7,5.

7. Verfahren zur Herstellung eines glasig-kristallinen Materials nach Anspruch 1, **dadurch gekennzeichnet, daß** ein Gemenge aus 15 - 45 Gew-% CaO, 40 - 45 Gew-% P₂O₅, 10 - 40 Gew-% ZrO₂ und 0,7 - 3,5 Gew-% Fluorid mit mehreren Haltestufen im Bereich von 400 bis 1500 °C bei 1550 bis 1650 °C aufgeschmolzen und das glasig-kristalline Material als spontan abgekühlte oder stufenweise abgekühlte Schmelze gewonnen wird.

8. Verfahren nach Anspruch 7, **dadurch gekennzeichnet, daß** dem Gemenge zusätzlich 0,1 bis 3 Gew-% Na₂O, MgO und/oder K₂O zugesetzt werden.

9. Verfahren nach Anspruch 7, **dadurch gekennzeichnet, daß** bis zu 15 Gew-% eines gemahlenen Glases eingebracht werden, das aus SiO₂, Al₂O₃ und MgO und gegebenenfalls CaO besteht und in seiner Zusammensetzung und seinen Eigenschaften einem Cordierit-Glas entspricht.

10. Verfahren nach Anspruch 7, **dadurch gekennzeichnet, daß** ein Gemenge aus 15 - 45 Gew-% CaO, 40 - 45 Gew-% P₂O₅, 10 - 40 Gew-% ZrO₂ und 0,7 - 3,5 Gew-% Fluorid mit mehreren Haltestufen im Bereich von 400 bis 1500°C bei 1550 bis 1650°C aufgeschmolzen und kontrolliert im Ofen mit einer Geschwindigkeit von 60°C/h bis 300°C/h mit zwei Haltestufen zwischen 1000 bis 1100°C und 800 bis 1000°C für 2 bis 10 h abgekühlt wird.

11. Verwendung eines glasig-kristallinen Materials nach Anspruch 1 zur Herstellung von Granulaten; oder von keramischen Formkörpern oder keramischen Folien mit dichter oder offenporiger Struktur.

## Claims

1. A glassy-crystalline material with low solubility on the basis of CaO, P₂O₅, ZrO₂ and fluoride which material comprises 15-45 % by weight CaO, 40-45 % by weight P₂O₅, 10-40 % by weight ZrO₂ and 0.7-3.5 % by weight of fluoride and contains apatite and calcium zirconium phosphate as main crystal phases and a glass phase as secondary component, the main crystal phases jointly making up at least 35 % by weight and the secondary components making up 5 to 15 % by weight, and all percentages being relative to the total weight of the glassy-crystalline material.

2. A material according to Claim 1 wherein the said material contains 23-39 % by weight CaO, 40-45 % by weight P₂O₅, 20-35 % by weight ZrO₂ and 1-3 % by weight of fluoride.

3. A material according to Claim 1 or 2 wherein the said material additionally contains 0.1 to 6 % by weight Na₂O, MgO and/or K₂O and corresponding phases as additional secondary component.

4. A material according to Claim 1 wherein the said material additionally contains a sodium zirconium phosphate phase.

5. A material according to Claim 1 wherein the said material is present in the form of a granulated material or a ceramically processed dense or porous body or a ceramic sheet.

6. A material according to Claim 1 wherein the said material has one or several of the following parameters
- a total solubility of 0.2 to 5.5 mg/l if the test is carried out in a 0.2 M TRIS HCl buffer solution at pH = 7.4, T = 37 °C, using a grain size fraction of 315-400 mm, the duration of the test being 120 h and the ratio of surface area (sample) to volume (solvent) being 5 cm⁻¹,
- a thermal coefficient of expansion between 1.4 and 8x10⁻⁶ degrees⁻¹ between 27 °C and 300, 400, 600 and 800 °C,
- stability in the pH range between 7.0 and 7.5.

7. A method for manufacturing a glassy-crystalline material according to Claim 1 wherein a mixture of 15-45 % by weight CaO, 40-45 % by weight P₂O₅, 10-40 % by weight ZrO₂ and 0.7-3.5 % by weight of fluoride is melted at 1550 to 1650 °C, the melting process including several holding stages in the range of 400 to 1500 EC, and the glassy-crystalline material is obtained in the form of a melted mass which is cooled down spontaneously or step by step.

8. A method according to Claim 7 wherein an additional 0.1 to 3 % by weight Na₂O, MgO and/or K₂O are added to the said mixture.

9. A method according to Claim 7 wherein up to 15 % by weight of a ground glass are added, which glass consists of SiO₂, Al₂O₃ and MgO and in optionally CaO and the composition and characteristics of which correspond to those of a cordierite glass.

10. A method according to Claim 7 which method comprises melting of a mixture of 15-45 % by weight CaO, 40-45 % by weight P₂O₅, 10-40 % by weight ZrO₂ and 0.7-3.5 % by weight of fluoride at 1550 to 1650 °C, the melting process including several holding stages in the range of 400 to 1500 °C, and cooling down the said mixture in the furnace in a controlled manner and at a rate of 60 °C/h to 300 °C/h, the cooling process lasting for 2 to 10 h and including two holding stages between 1000 and 1100 °C and between 800 and 1000 °C.

11. The use of a glassy-crystalline material according to Claim 1 for manufacturing granulated materials; or ceramic bodies or ceramic sheets having a dense or open-pore structure.

## Revendications

1. Matériau vitreux-cristallin avec une faible solubilité, à base de CaO, P₂O₅, ZrO₂ et fluorure, **caractérisé en ce qu'**il contient 15 à 45 % en poids de CaO, 40 à 45 % de P₂O₅, 10 à 40 % en ZrO₂ et 0,7 à 3,5 % en poids de fluorure, et contient comme phase cristalline principale, l'apatite et le phosphate de calcium-zirconium et comme constituant secondaire, une phase vitreuse, où les phases cristallines principales s'élèvent globalement, à au moins 35 % en poids et les constituants secondaires s'élèvent de 5 à 15 % en poids et où toutes les données en pourcentage se rapportent au poids total du matériau vitreux-cristallin.

2. Matériau selon la revendication 1, **caractérisé en ce qu'**il contient 23 à 39 % en poids de CaO, 40 à 45 % en poids de P₂O₅, 20 à 35 % en poids de ZrO₂ et 1 à 3 % en poids de fluorure.

3. Matériau selon la revendication 1 ou 2, **caractérisé en ce qu'**il contient en outre, 0,1 à 6 % en poids de Na₂O, de MgO et/ou de K₂O et les phases appropriées comme constituant secondaire supplémentaire.

4. Matériau selon la revendication 1, **caractérisé en ce qu'**il contient en outre, une phase phosphate de sodium-zirconium.

5. Matériau selon la revendication 1, **caractérisé en ce qu'**il se présente sous la forme d'un granulé ou sous la forme d'un corps moulé dense ou poreux et traité comme une céramique ou sous la forme d'une feuille céramique.

6. Matériau selon la revendication 1, **caractérisé en ce qu'**il présente un ou plusieurs des paramètres suivants :
- solubilité totale de 0,2 à 5,5 mg/litre, lorsque l'essai est réalisé dans une solution tampon TRIS-HCl 0,2 M à pH 7,4, T = 37°C, sur une fraction des grains 315 à 400 µm, pendant 120 heures à un rapport surface (échantillon) à volume (solvant) de 5 cm⁻¹,
- coefficient d'allongement thermique entre 1,4 et 8 x 10⁻⁶ degré⁻¹ entre 27°C et 300, 400, 600 et 800°C,
- stabilité dans l'intervalle de pH allant de 7,0 à 7,5.

7. Procédé de préparation d'un matériau vitreux-cristallin selon la revendication 1, **caractérisé en ce que** l'on fait fondre un mélange de 15 à 45 % en poids de CaO, 40 à 45 % en poids de P₂O₅, 10 à 40 % en poids de ZrO₂ et 0,7 à 3,5 % en poids de fluorure, avec plusieurs étapes de maintien, dans l'intervalle allant de 400 à 1500°C, jusqu'à 1550 à 1650°C, et **en ce que** le matériau vitreux-cristallin est obtenu comme masse fondue refroidie spontanément ou refroidie par étape.

8. Procédé selon la revendication 7, **caractérisé en ce que** l'on ajoute en outre au mélange, 0,1 à 3 % en poids de Na₂O, de MgO et/ou de K₂O.

9. Procédé selon la revendication 7, **caractérisé en ce que** l'on introduit jusqu'à 15 % en poids d'un verre broyé, qui consiste en SiO₂, Al₂O₃ et MgO, et le cas échéant CaO, et qui correspond par sa composition et ses propriétés, à un verre cordiérite.

10. Procédé selon la revendication 7, **caractérisé en ce que** l'on fait fondre un mélange de 15 à 45 % en poids de CaO, de 40 à 45 % en poids de P₂O₅, de 10 à 40 % en poids de ZrO₂ et de 0,7 à 3,5 % en poids de fluorure, avec plusieurs étapes de maintien dans l'intervalle de 400 à 1500°C, jusqu'à 1550 à 1650°C, et il est refroidi de manière contrôlée dans un four, avec une vitesse de 60°C/h à 300°C/h, avec deux étapes de maintien entre 1000 et 1100°C et entre 800 et 1000°C, pendant 2 à 10 heures.

11. Utilisation d'un matériau vitreux-cristallin selon la revendication 1, pour la préparation de granulés, ou d'articles moulés céramiques ou de feuilles céramiques avec structure dense ou à pores ouverts.
